# EUROPEAN PATENT SPECIFICATION

(11) **EP 4 241 154 B1**
(45) Date of publication and mention of the grant of the patent: **20.05.2026**
(21) Application number: 20804344.8
(22) Date of filing: 03.11.2020
(51) Int. Cl.: G06F 1/16, A61L 9/12, G06F 3/01

(54) **WELLNESS AND PRODUCTIVITY COMPUTER ACCESSORY DEVICE**
COMPUTERZUBEHÖRVORRICHTUNG FÜR WOHLBEFINDEN UND PRODUKTIVITÄT
DISPOSITIF ACCESSOIRE D'ORDINATEUR DE BIEN-ÊTRE ET DE PRODUCTIVITÉ

(43) Date of publication of application: 13.09.2023
(73) Proprietor: HCOMM S.r.l., 63028 Montefalcone Appennino FM (IT)
(72) Inventor: CIMADAMORE, Anna Luisa, 63900 Fermo FM (IT)
(74) Representative: Manna, Sara
(86) International application number: PCT/IB2020/060290
(87) International publication number: WO 2022/096915

(56) References cited:
- WO-A2-2009/098693
- KR-A- 20190 074 495
- US-A1- 2018 144 033
- US-A1- 2019 212 787
- US-A1- 2020 208 867
- US-A1- 2020 215 213

## Description

### Background of the invention

Nowadays, both office work and teleworking force individuals to spend more and more time in front of the computer or PC screen.

Spending so much time in front of the PC screen can have negative consequences on psychophysical efficiency and productivity, and in particular case can even damage workers' health.

As way of example, every user of a PC as experienced, at least once, how difficult it is to maintain good working efficiency for a long period of time. Tiredness, exhaustion after a long meeting, conflicts between colleagues, disappointed expectations, as well as a worry or a sleepless night, can raise levels of stress, anger and anxiety, and make it difficult to concentrate on work. In addition, many scientific studies have shown that concentration and focus on work activities, if interrupted, take from 10 to 23 minutes to return to the same pre-interruption level. During office work and teleworking, interruptions - a phone call, a colleague which requires attention - are very frequent.

On the other side, also the workers' body is often negative affected by working at the computer. Incorrect postures assumed at workstations can cause painful or inflammatory states, as well as headaches, blood circulation problems and fatigue. Such problems are compounded by the fact that in a prolonged demanding work, the state of tiredness and inefficiency, cerebral and osteo-muscular, is not felt by the user.

Furthermore, in the presence of working methods which provide sharing of desks, workstations and IT equipment, the probability of coming into contact with pathogens or viruses from previous users is very high.

KR20190074495A discloses an electronic frame system, which comprises an electronic frame in combination with a wearable device capable of being worn on a user's body. The electronic frame includes a camera, a speaker, a display unit, a control unit and a communication unit. The wearable device is capable of measuring and storing user's pulse and body temperature and transmitting such data wirelessly to the electronic frame. The electronic frame outputs an image suitable for the user on the display unit, based on the pulse data and the body temperature data transmitted by the wearable device.

### Summary of the invention

The object of the present invention is providing an accessory device for a computer, configured to take care of the psychophysical users' health, avoiding the previously mentioned drawbacks.

The accessory device is configured to work according to completely automated way, in order to not require actions by the user.

In particular, the accessory device comprises a high-tech external "shell" or container, which contains operative modules provided with artificial intelligence.

Such modules comprise sensors configured to detect user's physical parameters and identify abnormal psychophysical states, as well as automated appliances configured to start treatment programs accordingly to the user's state.

According to preferred embodiments of the invention, here are some particularly significant operational possibilities of the accessory device:
- providing interactive technologies, developed by Al systems, designed to support the user during his working day in order to realize: reduction of stress and anger, improvement of concentration, or stimulation of creativity, and many others;
- acting proactively by blocking and discreetly reporting unwanted outages to the user;
- monitoring the user's postural status and, in the presence of prolonged incorrect positions, inviting the user to correct the position;
- monitoring the psychophysical condition of the user and, if needed, inviting the user to follow a personalized protocol to restore well-being, concentration and efficiency.

In other words, the device of the invention is configured to monitor the psychophysical state of the user, by detecting physical parameters such as body temperature, heart rate, breathing rate, or posture. Through the processing of one or more of said parameters, the device can also determine an emotional feedback from the user. The processing of such parameters according to predetermined algorithms allows to estimate the overall psychophysical situation of the user, highlighting states of fatigue, stress and/or probability of flu.

Advantageously, the accessory device according to the invention allows the user to bring their mind into "mindfulness" mode in a few minutes.

Advantageously, preferred embodiments of the invention allow the user to maintain a state of general well-being by implementing health suggestions based on memorized therapeutic plans. For example, if a user needs to follow certain therapies and/or has particular pathologies that require action, the device can send messages to the user at scheduled times, reminding the assumption of a certain dose of drugs.

According to a preferred embodiment, the invention is equipped with a UV-C LED light disinfectant lamp that eliminates 99% of viruses and bacteria, sanitizing keyboard, mouse, desk chair and all objects near the accessory device, avoiding the risk of any contagion.

Therefore, the computer accessory device according to the invention allows preventing the spread of pathogens and virus in working environments.

Advantageously, the device according to the invention automatically combats pathogens and viruses and prevents damages to physical health of the user. The invention is defined by the appended claims.

### Brief description of the drawings

Reference will be made to the Figures of the annexed drawings, wherein:
- Figure 1 shows a perspective frontal view of a preferred embodiment of a device according to the present invention;
- Figure 2 shows a perspective frontal view of the device of Figure 1, without a frontal portion of a containment shell;

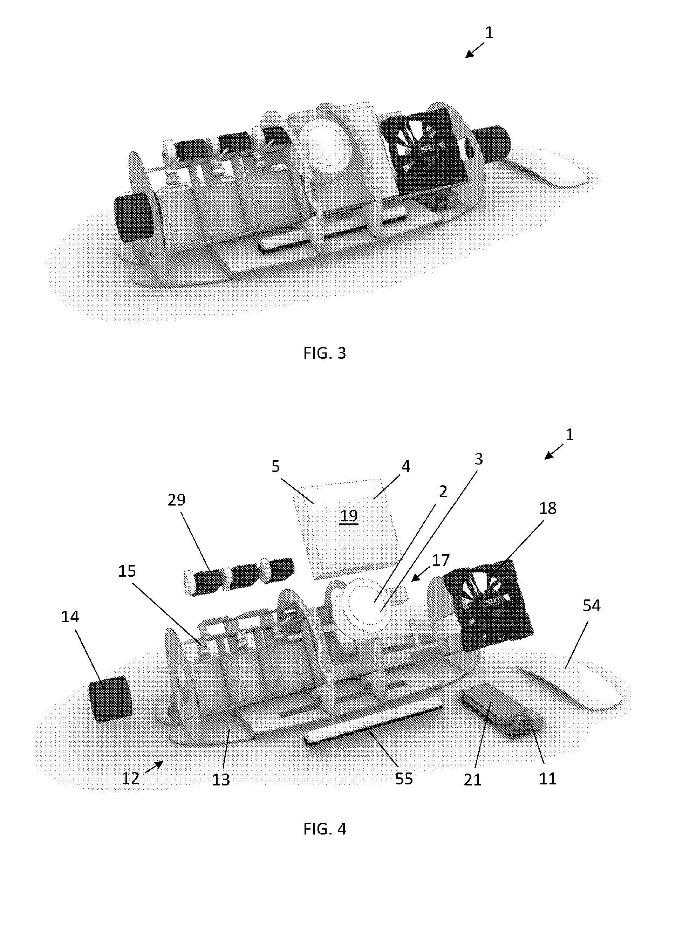

- Figure 3 shows a perspective frontal view of the device of Figure 1, without frontal and rear portions of the containment shell;
- Figure 4 shows a perspective frontal exploded view of the device of Figure 1, without frontal and rear portions of containment shell;

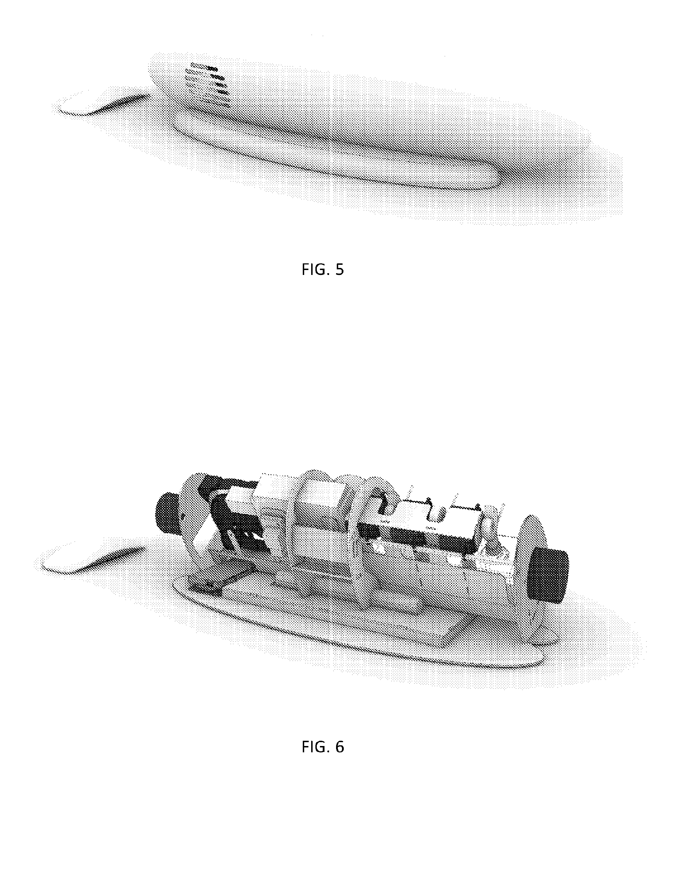

- Figure 5 shows a perspective rear view of the device of Figure 1;
- Figure 6 shows a perspective rear view of the device of Figure 1, without the rear portion of the containment shell; and
- Figure 7 shows a partial perspective frontal view of a preferred embodiment of a computer comprising a device according to the present invention, which containment shell is transparent.

The above-mentioned Figures are to be meant exclusively by way of example and not for limitative purposes.

### Detailed description of preferred embodiments of the invention

With reference to Figures from 1 to 6, an accessory device for a computer according to a preferred embodiment of the present invention is globally denoted by 1.

The accessory device 1 comprises a plurality of modules 10 for monitoring and improving a user's work experience at the computer.

The modules 10 comprise one or more sensors 20 configured to detect physical data of the user, in particular a video camera 2 for detecting images of the user and/or a microphone 3 for detecting sounds from the user.

The modules 10 comprise also a speaker 14 for the emission of predetermined sounds, preferably an extractable speaker, at least a diffuser 15 of essence, and at least a respective tank 6 comprising said essence.

For the delivery of the essences, oner or more servomotors 29 for actuate the diffuser are comprised in the device 1.

Furthermore, the modules 10 comprise a memory unit 4, wherein reference data, associated to the user, are stored, together with instructions for operating the speaker 14 and diffuser 15. Such instructions are stored in association to a plurality of possible results of a comparison between data detected by the sensors 20 and the reference data.

Also, the modules 10 comprise a CPU or processing unit 5, programmable according to user needs.

The processing unit 5 is adapted to receive data detected by the sensors 20 and compare them to reference data of the user, stored in the memory unit 4.

After the comparison, the processing unit 5 is adapted to select instructions for operating speaker 14 and diffuser 15 - as well as other sensors eventually comprised in the device 1, according to results of the comparison - and operate said speaker 14 and diffuser 15 accordingly.

As way of example, in the memory unit 4, data about a breathing rate of the user in a rest and relaxed condition can be stored. Also, instructions for operating speaker 14 and diffuser 15 are stored, associated to possible results of a comparison between data detected by a microphone 3 and the reference data. When the microphone 3 detects data referred to the breath rate of the user, it sends such data to the processing unit 5. The processing unit 5 compares the data received from the speaker 14 to the reference data stored in the memory unit 4, and selects instructions for operating the speaker 14 and the diffuser 15 according to the result of the comparison. For example, if the breathing rate detected by the microphone 3 is higher than the breathing rate of the user in a rest and relaxed condition, the instructions associated to such result is that of reproducing a relaxing melody (also stored in the memory unit 4) by means of the speaker 14 and activate the diffuser 15 for delivery a relaxing essence, stored in the tank 6. Then, the processing unit 5 controls the operation of the speaker 14 and the diffuser 15 according to such instructions.

The accessory device 1 further comprises connection devices 11 configured to connect at least one of the modules 10 to the computer 100.

Basically, connection devices 11 guarantee the integrability of the device 1 with every kind of computer (both fixed and portable). They can comprise sockets - LAN, USBs, HDMI - and ports, like a UDSB port. The connection devices 11 are preferably configured to acquire electric power from the computer the device 1 is associated to, in order to avoid the presence of a battery in the device 1. According to another embodiment of the invention, a battery is comprised in the accessory device.

In other words, the device 1 can be connected to any computer or PC, preferably via a USB cable. The device 1 can further comprise a processor 19 ('miniaturized PC') inside the casing 2, preferably associated to the processing unit 5, which can be expanded into a configuration that does not need an external PC, by connecting directly to monitor, keyboard and mouse. An electronic board 21 connects all the devices, sensors, servomotors included in the device 1, to the processor 19.

The processing unit 5 can be provided with an Artificial Intelligence (AI) system trained for recognizing emotional states, postures, level of fatigue, symptoms of a possible pathology (for example, cooling, fever, breathing difficulties) and selecting instructions for operating the device 1 according to the detected states.

The Al system could be a self-configuring system through a series of questionnaires that the user is invited to fill in during the first installation of the accessory device, and that the Al system uses to make a first calibration of its HW / SW technologies.

All the modules 10 and the connection devices 11 are contained in an external casing 2, preferably configured like a shell. The casing 2 comprises support elements 12 for placing the accessory device 1 on a surface S in a stable manner.

Preferably, the casing 2 is configured to make the device 1 be "portable", and comfortably carried and inserted under any kind of monitor (desktop PC).

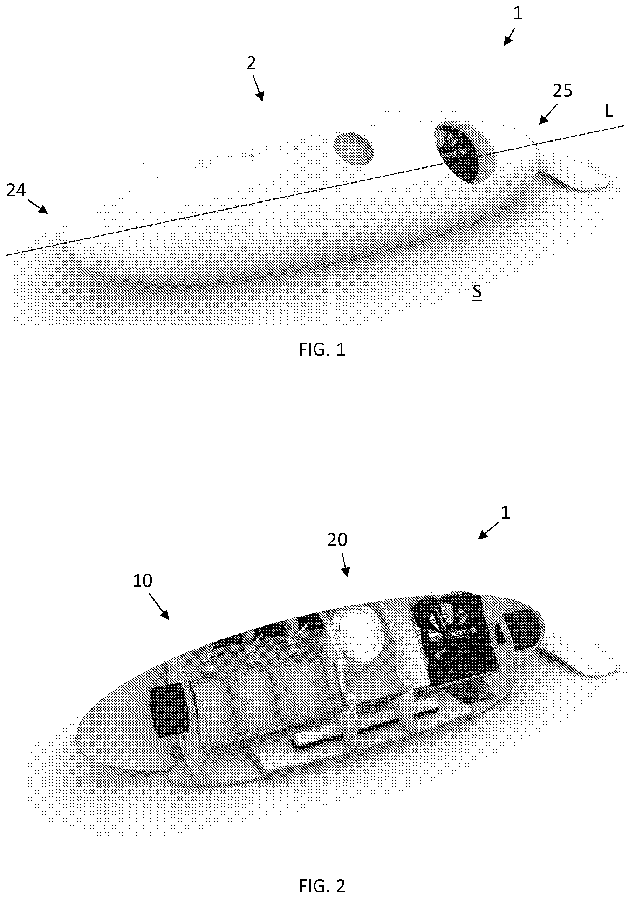

The casing 2 is preferably formed like a shell having a rear and a front portion, more preferably having a pedestal 13 for supporting on a working surface S. The shell can have an elongated form according to a longitudinal direction L, which is a horizontal direction in use, preferably with a circular or elliptical section which reduces at the longitudinal terminal end 24, 25 thereof (Figure 1).

According to a preferred embodiment of the invention, the sensors 20 are configured to detect at least one of the following data: body temperature, heart rate, breathing rate and position of the user's body.

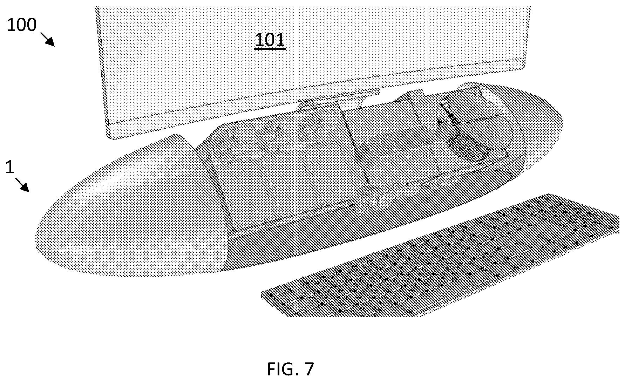

Preferably, when the sensors 20 are configured to detect the position of user's body, the memory unit 4 is adapted to store data about correct positions of user's body and the processing unit 5 is programmed to compare the detected position of user's body with the correct positions of the user's body. If the detected position is different than the memorized correct positions, the processing unit 5 is further programmed to send a predetermined control signal to the computer 100, which the accessory device 1 is associated to (see Figure 7). The predetermined control signal is configured to make a computer screen 101 display a message which content is an invitation to correct user's position.

Similarly, the memory unit 4 can be adapted to store data about a therapeutic plan of the user. Accordingly, the processing unit 5 can be programmed to send another predetermined control signal to the computer 100, such signal being configured to make the computer screen 101 display a message which content is a reminder about said therapeutic plan.

Furthermore, preferably, the modules 10 comprise disinfectant devices 7, configured to provide disinfectant substances or light beams. Preferably, the disinfectant devices 7 comprises a UV-C LED light disinfectant lamp, which can be configured to eliminate 99% of viruses and bacteria by sterilizing keyboard, mouse, desk chair and various objects in its vicinity in a few seconds.

Preferably, the device 1 could be equipped with a first central UV-C LED lamp 55 for disinfecting the keyboard, and a second lateral UV-C LED lamp 54 for disinfecting the mouse or other objects on the desk.

According to preferred embodiments of the invention, the modules 10 comprise an air management system 18, and/or a phosphenic lamp 17 with a camera and a microphone attached thereto.

In particular, as regarding the central processing unit 4, the electronic management and control board is designed and built on the related PCB.

In the memory unit 4 a basic series of treatments can be stored. A larger list of additional therapies, courses and specific treatments on cloud could be available to the user, and downloaded. Each treatment is carried out by implementing programmed multisensorial stimuli: essences, sounds, images, phosphenic light, audio voices (where each frequency of each stimulus has been harmonized with the others). The treatments induce in the user effects of relaxation, reduction of a state of anger/disappointment, as well as increased concentration.

Treatments and courses have the aim to improve general skills and performance of workers (e.g. learning and mental performance, quick reading, mind maps for work organization, but also coaching mini-courses, development of relation skills, intuition, leadership strategies with NLP techniques, etc.). Also, personalized courses (thematic courses) can be provided, which implement specific contents, also tailor-made upon request by the client company (e.g. a specialization or training course for laboratory technicians, or for operators in a specific sector, etc.), or are aimed at changing bad habits, improve personal performance, strengthening one's resources, and many others.

The treatments can be divided into "shots" (single treatments of about thirty minutes to be carried out in extra working hours) and "mini" sessions (quick treatments of a few minutes to be carried out during working hours for emergencies which can be followed by post-treatments to strengthen the results).

According to another embodiment, the device 1 can be associated to a headband, configured to provide electromagnetic stimuli to the user according to the instructions of the processing unit 5 (for example, when a stress condition of the user is detected by the sensors 20).

The present invention has been described so far with reference to preferred embodiments, which are intended to be combined if compatible. It is intended that there may be other embodiments which refer to the same inventive concept and fall within the scope of the appended claims.

## Claims

1. An accessory device (1) for a computer (100), comprising:
- a plurality of modules (10) for monitoring and improving a user's work experience at the computer (100), said modules (10) comprising:
• one or more sensors (20) configured to detect data of the user;
• a speaker (14) for the emission of predetermined sounds;
• a diffuser (15) of essences, and a tank (6) comprising said essences;
• a memory unit (4), wherein reference data, associated to the user, are stored, and wherein instructions for operating said speaker (14) and diffuser (15) are stored,
said instructions being stored in association with a plurality of predetermined possible results of a comparison between data detected by said one or more sensors (20) and said reference data;
• a processing unit (5), adapted to:
receive data detected by said one or more sensors (20), compare them to said reference data, select instructions for operating said speaker (14) and diffuser (15) according to the result of the comparison, and operate said speaker (14) and diffuser (15) according to the selected instructions;
- connection devices (11) configured to connect at least one of said plurality of modules (10) to the computer (100);
- an external casing (2), configured to contain said plurality of modules (10) and said connection devices (11), further comprising support elements (12) for placing the accessory device (1) on a surface (S).
wherein said one or more sensors (20):
comprise a video camera (2) for detecting images of the user,
comprise a microphone (3) for detecting sounds from the user, and
are further configured to detect the following user's data: body temperature, heart rate, breathing rate, position of the user's body.

2. The accessory device (1) according to any of the preceding claims, wherein:
said one or more sensors (20) are configured to detect the position of user's body;
said memory unit (4) is adapted to store data about correct positions of user's body; and
said processing unit (5) is programmed to compare a detected position of user's body with the correct positions of the user's body, and
if the detected position is different than the correct positions, said processing unit (5) being further programmed to send a first predetermined control signal to the computer (100), said first predetermined control signal being configured to make the computer screen (101) display a message which content is an invitation to correct user's position.

3. The accessory device (1) according to any of the preceding claims, wherein:
said memory unit (4) is adapted to store data about a therapeutic plan of the user; and
said processing unit (5) is programmed to send a second predetermined control signal to the computer (100), said second predetermined control signal being configured to make the computer screen (101) display a message which content is a reminder about said therapeutic plan.

4. The accessory device (1) according to any of the preceding claims, configured to acquire electric power from the computer (100) by means of said connection devices (11).

5. The accessory device (1) according to the preceding claim, wherein said connection devices (11) comprises a USB port.

6. The accessory device (1) according to any of the preceding claims, wherein said plurality of modules (10) comprises disinfectant devices (7), configured to provide disinfectant light beams.

7. The accessory device (1) according to claim 6, wherein said disinfectant devices (7) comprise a UV-C LED light disinfectant lamp (55, 54).

8. The accessory device (1) according to any of the preceding claims, wherein said support elements (12) comprise a pedestal (13).

9. Computer (100) comprising an accessory device (1) according to any of the preceding claims.

## Patentansprüche

1. Zubehörvorrichtung (1) für einen Computer (100), umfassend:
- eine Vielzahl von Modulen (10) zum Überwachen und Verbessern der Arbeitserfahrung eines Benutzers an dem Computer (100), die Module (10) umfassend:
• einen oder mehrere Sensoren (20), die konfiguriert sind, um Daten des Benutzers zu erfassen;
• einen Lautsprecher (14) für die Ausgabe zuvor bestimmter Töne;
• einen Diffusor (15) für Essenzen und einen Behälter (6), umfassend die Essenzen;
• eine Speichereinheit (4), wobei Referenzdaten, die dem Benutzer zugeordnet sind, gespeichert sind, und wobei Anweisungen zum Betreiben des Lautsprechers (14) und des Diffusors (15) gespeichert sind,
wobei die Anweisungen in Zuordnung zu einer Vielzahl von zuvor bestimmten möglichen Ergebnissen eines Vergleichs zwischen Daten, die durch den einen oder die mehreren Sensoren (20) erfasst werden, und den Referenzdaten gespeichert sind;
• eine Verarbeitungseinheit (5), die angepasst ist zum:
Empfangen von Daten, die durch den einen oder die mehreren Sensoren (20) erfasst werden, Vergleichen dieser mit den Referenzdaten, Auswählen von Anweisungen zum Betreiben des Lautsprechers (14) und des Diffusors (15) gemäß dem Ergebnis des Vergleichs und Betreiben des Lautsprechers (14) und des Diffusors (15) gemäß den ausgewählten Anweisungen;
- Verbindungsvorrichtungen (11), die konfiguriert sind, um mindestens eines der Vielzahl von Modulen (10) mit dem Computer (100) zu verbinden;
- ein externes Gehäuse (2), das konfiguriert ist, um die Vielzahl von Modulen (10) und die Verbindungsvorrichtungen (11) zu enthalten, ferner umfassend Stützelemente (12) zum Platzieren der Zubehörvorrichtung (1) auf einer Oberfläche (S).
wobei der eine oder die mehreren Sensoren (20):
eine Videokamera (2) zum Erfassen von Bildern des Benutzers umfassen,
ein Mikrofon (3) zum Erfassen von Geräuschen des Benutzers umfassen und
ferner konfiguriert sind, um die folgenden Daten des Benutzers zu erfassen: Körpertemperatur, Herzfrequenz, Atemfrequenz, Position des Körpers des Benutzers.

2. Zubehörvorrichtung (1) nach einem der vorstehenden Ansprüche, wobei:
der eine oder die mehreren Sensoren (20) konfiguriert sind, um die Position des Körpers des Benutzers zu erfassen;
die Speichereinheit (4) angepasst ist, um Daten über korrekte Positionen des Körpers des Benutzers zu speichern; und
die Verarbeitungseinheit (5) programmiert ist, um eine erfasste Position des Körpers des Benutzers mit den korrekten Positionen des Körpers des Benutzers zu vergleichen und
falls sich die erfasste Position von den korrekten Positionen unterscheidet, die Verarbeitungseinheit (5) ferner programmiert ist, um ein erstes zuvor bestimmtes Steuersignal an den Computer (100) zu senden, wobei das erste zuvor bestimmte Steuersignal konfiguriert ist, um den Computerbildschirm (101) zu veranlassen, eine Nachricht anzuzeigen, deren Inhalt eine Aufforderung ist, die Position des Benutzers zu korrigieren.

3. Zubehörvorrichtung (1) nach einem der vorstehenden Ansprüche, wobei:
die Speichereinheit (4) angepasst ist, um Daten über einen Therapieplan des Benutzers zu speichern; und
die Verarbeitungseinheit (5) programmiert ist, um ein zweites zuvor bestimmtes Steuersignal an den Computer (100) zu senden, wobei das zweite zuvor bestimmte Steuersignal konfiguriert ist, um den Computerbildschirm (101) zu veranlassen, eine Nachricht anzuzeigen, deren Inhalt eine Erinnerung an den Therapieplan ist.

4. Zubehörvorrichtung (1) nach einem der vorstehenden Ansprüche, die konfiguriert ist, um elektrische Leistung von dem Computer (100) mittels der Verbindungsvorrichtungen (11) zu beziehen.

5. Zubehörvorrichtung (1) nach dem vorstehenden Anspruch, wobei die Verbindungsvorrichtungen (11) einen USB-Anschluss umfassen.

6. Zubehörvorrichtung (1) nach einem der vorstehenden Ansprüche, wobei die Vielzahl von Modulen (10) Desinfektionsmittelvorrichtungen (7) umfasst, die konfiguriert sind, um desinfizierende Lichtstrahlen bereitzustellen.

7. Zubehörvorrichtung (1) nach Anspruch 6, wobei die Desinfektionsmittelvorrichtungen (7) eine UV-C-LED-Licht-Desinfektionslampe (55, 54) umfassen.

8. Zubehörvorrichtung (1) nach einem der vorstehenden Ansprüche, wobei die Stützelemente (12) einen Sockel (13) umfassen.

9. Computer (100), umfassend eine Zubehörvorrichtung (1) nach einem der vorstehenden Ansprüche.

## Revendications

1. Dispositif accessoire (1) destiné à un ordinateur (100), comprenant :
- une pluralité de modules (10) permettant de surveiller et d'améliorer une expérience de travail d'un utilisateur au niveau de l'ordinateur (100), lesdits modules (10) comprenant :
• un ou plusieurs capteurs (20) configurés pour détecter des données de l'utilisateur ;
• un haut-parleur (14) pour l'émission de sons prédéterminés ;
• un diffuseur (15) d'essences, et un réservoir (6) comprenant lesdites essences ;
• une unité de mémoire (4), dans laquelle des données de référence, associées à l'utilisateur, sont stockées, et dans laquelle des instructions permettant de faire fonctionner ledit haut-parleur (14) et ledit diffuseur (15) sont stockées,
lesdites instructions étant stockées en association avec une pluralité de résultats possibles prédéterminés d'une comparaison entre des données détectées par ledit ou lesdits capteurs (20) et lesdites données de référence ;
• une unité de traitement (5), adaptée pour :
recevoir des données détectées par ledit ou lesdits capteurs (20), les comparer auxdites données de référence, sélectionner des instructions permettant de faire fonctionner ledit haut-parleur (14) et ledit diffuseur (15) selon le résultat de la comparaison, et faire fonctionner ledit haut-parleur (14) et ledit diffuseur (15) selon les instructions sélectionnées ;
- des dispositifs de connexion (11) configurés pour connecter au moins l'un parmi ladite pluralité de modules (10) à l'ordinateur (100) ;
- un boîtier externe (2), conçu pour contenir ladite pluralité de modules (10) et lesdits dispositifs de connexion (11), comprenant en outre des éléments de support (12) permettant de placer le dispositif accessoire (1) sur une surface (S).
dans lequel ledit ou lesdits capteurs (20) :
comprennent une caméra vidéo (2) permettant de détecter des images de l'utilisateur,
comprennent un microphone (3) permettant de détecter des sons provenant de l'utilisateur, et
sont configurés en outre pour détecter des données d'utilisateur suivantes : température corporelle, fréquence cardiaque, fréquence respiratoire, position du corps de l'utilisateur.

2. Dispositif accessoire (1) selon l'une quelconque des revendications précédentes, dans lequel :
ledit ou lesdits capteurs (20) sont configurés pour détecter la position du corps de l'utilisateur ;
ladite unité de mémoire (4) est adaptée pour stocker des données concernant des positions correctes du corps de l'utilisateur ; et
ladite unité de traitement (5) est programmée pour comparer une position détectée du corps de l'utilisateur aux positions correctes du corps de l'utilisateur, et
si la position détectée est différente des positions correctes, ladite unité de traitement (5) est en outre programmée pour envoyer un premier signal de commande prédéterminé à l'ordinateur (100), ledit premier signal de commande prédéterminé étant configuré pour amener l'écran d'ordinateur (101) à afficher un message dont le contenu est une invitation à corriger la position de l'utilisateur.

3. Dispositif accessoire (1) selon l'une quelconque des revendications précédentes, dans lequel :
ladite unité de mémoire (4) est adaptée pour stocker des données concernant un plan thérapeutique de l'utilisateur ; et
ladite unité de traitement (5) est programmée pour envoyer un second signal de commande prédéterminé à l'ordinateur (100), ledit second signal de commande prédéterminé étant configuré pour amener l'écran d'ordinateur (101) à afficher un message dont le contenu est un rappel concernant ledit plan thérapeutique.

4. Dispositif accessoire (1) selon l'une quelconque des revendications précédentes, configuré pour acquérir une puissance électrique provenant de l'ordinateur (100) au moyen desdits dispositifs de connexion (11).

5. Dispositif accessoire (1) selon la revendication précédente, dans lequel lesdits dispositifs de connexion (11) comprennent un port USB.

6. Dispositif accessoire (1) selon l'une quelconque des revendications précédentes, dans lequel ladite pluralité de modules (10) comprend des dispositifs désinfectants (7), configurés pour fournir des faisceaux de lumière désinfectants.

7. Dispositif accessoire (1) selon la revendication 6, dans lequel lesdits dispositifs désinfectants (7) comprennent une lampe désinfectante à lumière DEL UV-C (55, 54).

8. Dispositif accessoire (1) selon l'une quelconque des revendications précédentes, dans lequel lesdits éléments de support (12) comprennent un socle (13).

9. Ordinateur (100) comprenant un dispositif accessoire (1) selon l'une quelconque des revendications précédentes.
